# EUROPEAN PATENT APPLICATION

(11) **EP 1 798 220 A1**
(43) Date of publication of application: **20.06.2007**
(21) Application number: 05077910.7
(22) Date of filing: 16.12.2005
(51) Int. Cl.: C07C 255/60, A61K 31/166, A61P 25/28

(54) **Dibenzene derivatives as calcium channel blockers**

(71) Applicant: Neuropharma S.A., 28760 Tres Cantos (Madrid) (ES)
(72) Inventor: Martínez Gil, Ana, 28004 Madrid (ES); Castro Morera, Ana, 28028 Madrid (ES); Medina Padilla, Miguel, 28029 Madrid (ES); Muñoz Ruiz, Pilar, 28923 Alcorcòn - Madrid (ES); Rubio Arrieta, Laura, 28024 Madrid (ES); García Palomero, Esther, 28911 Leganés - Madrid (ES); De Austria, Celia, 28030 Madrid (ES)
(74) Representative: ABG Patentes, S.L.

(57) **Abstract**

The invention is directed to a compound of formula (I) 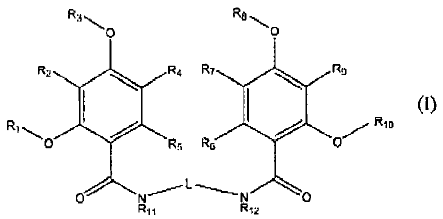 Having VDCC blocking activity. These compounds are useful for the treatment of a series of human diseases and conditions, especially cognitive or neurodegenerative diseases or conditions.

## Description

### FIELD OF THE INVENTION

This invention is related to a new family of synthetic compounds and to their use in the treatment of cognitive or neurodegenerative diseases, disorders or conditions.

### BACKGROUND OF THE INVENTION

Calcium Ca²⁺ concentrations and specially their fluctuation in different cellular subcompartments seem to be a universal signaling system, thus regulating most of the cellular functions, from contraction to gene expression through cell death.

The calcium ion is one of the most important elements in the physiological equilibrium of cells, acting not only as a neurotransmitter, but also as a second messenger. In order to control levels of calcium inside and outside cells, they are provided with different types of calcium channels. These channels control the calcium influx through the membrane where they are located, and they can be modulated by voltage changes or by ligands. The voltage-dependant Ca²⁺ channels (VDCCs) are an important type of calcium channels which are very numerous in cells with electrophysiological activity, such as neurons and muscle fibres cells. They consist of five subunits encoded by different groups of genes and designated as α₁ (the channel forming subunit), α₂δ, β, γ. The complex is provided with several sites for N-glycosylation and AMP-dependant protein kinases phosphorylation. When calcium enters the cytoplasma, it can bind different modulating proteins, to provoke diverse sequences of steps which in turn lead to different physiological changes. Calcium signaling pathways have several critical functions, such as nerve impulse transmission, muscle contraction, hormones secretion and constriction/relaxation of blood vessels.

However, an uncontrolled level of calcium can lead to different negative effects, such as neuronal excitotoxicity and other forms of cell death (*Mechanisms of calcium-related cell death,* Orrenius et al., Adv Neurol. 1996;71:137-49). Excitotoxicity is an excessive release of neurotransmitters which damages cells of the CNS (*Inciting excitotoxic cytocide among central neurons,* Olney J.W., Adv Exp Med Biol. 1986;203:631-45) and is often attributed to glutamate. An excessive synaptic release of glutamate can lead to the disregulation of Ca²⁺ homeostasis. Glutamate activates postsynaptic ionotropic receptors, such as NMDA or AMPA, which open their associated ionic channels to allow the influx of Ca²⁺ and other ions. Although the exact mechanism by which Ca²⁺ mediates excitotoxicity seems not to have been determined with complete certainty, some authors have hypothesized that it occurs following the activation of distinct signaling cascades downstream from key points of Ca²⁺ entry at synapses (Molecular mechanism of calcium-dependent neurodegeneration in excitotoxicity, Arundine M. and Tymianski M., Cell Calcium; 2003; 34 (4-5):325-327).

There are a lot of evidence that excitotoxicity may play a role in certain neuropathological events, such as neuronal death in stroke and ischemia, and in neurodegenerative diseases, such as Huntington's Disease (HD), Parkinson's Disease (PD) or Alzheimer's Disease (AD) (The role of excitotoxicity in neurodegenerative disease: implications for therapy, Doble A., Pharmacol Ther. 1999;81(3):163-221; Glutamate-mediated excitotoxicity and neurodegeneration in Alzheimer's disease, Hynd MR, Scott HL, Dodd PR., Neurochem. Int. 2004;45(5):583-95). Changes in the number and structure of VDCCs occur in the brain during the aging process, and these changes arc closely associated with several development functions of cells. Thus, VDCCs could be involved in increasing the vulnerability of the CNS cells to excitotoxicity with age (Decreased G-Protein-Mediated Regulation and Shift in Calcium Channel Types with Age in Hippocampal Cultures, Landfield ct al., J. Neurosci., 1999; 19(19):8674-8684).

Furthermore, the accumulation of amyloid-β-protein (Aβ) in the brain is a characteristic event in the pathology of Alzheimer's Disease. The processing of amyloid protein precursor (APP) results in the production of Aβ peptides with different numbers of aminoacids in their chains. These peptides have been found to be toxic to cells in culture because they disrupt calcium homeostasis in human cortical neurons (β-Amyloid peptides destabilize calcium homeostasis and render human cortical neurons vulnerable to excitotoxicity, Mattson MP et al., J Neurosci. 1992;12(2):376-89), and this process may be mediated in part by the opening of certain VDCCs (Amyloid beta protein potentiates Ca2+ influx through L-type voltage-sensitive Ca2+ channels: a possible involvement of free radicals, Ueda K et al., J Neurochem. 1997;68(1):265-71). Another physiological change in AD is an increase in acetylcholinesterase (AchE) activity around the amyloid accumulations, which leads to a loss of efficiency in both cholinergic and non-cholinergic neurons in the brain. The influx of calcium through certain VDCCs seems to have an effect on AChE expression because drugs acting as blockers of these Ca2+ channels, such as nifedipine, resulted in a decrease of AChE expression in cultured cells (The amyloid beta-protein of Alzheimer's disease increases acetylcholinesterase expression by increasing intracellular calcium in embryonal carcinoma P19 cells, Sberna G. et al., J Neurochem. 1997;69(3):1177-84).

Other publications have related disorders in the levels of Ca²⁺ in nerve cells with other diseases and disorders, especially cognitive and neurodegenerative diseases and disorders. This is the case, for example, of WO2005/097779, wherein the control of Ca²⁺ concentration in cells has been related to diseases such as stroke, anxiety (such as panic disorder, obsessive-compulsive disorder, post-traumatic stress syndrome), epilepsy, head trauma, migraine, chronic pain (such as cancer pain, inflammatory pain conditions related to osteoarthritis, rheumatoid arthritis and fibromyalgia), neuropathic pain (such as diabetic peripheral neuropathy, post-herpetic neuralgia, trigeminal neuralgia, cancer pain and AIDS related neuropathy) and acute pain (such as nocicceptive pain and post-operative pain., schizophrenia, depression, psychoses, drug and alcohol addiction, and neurodegenerative disorders (such as Parkinson's Disease, Alzheimer's Disease, multiple sclerosis, neuropathies, Huntington's Disease and amyotrophic lateral sclerosis (ALS)).

Therefore, taking into account that Ca²⁺ seems to have a direct implication in a series of important human diseases and disorders, especially cognitive and neurodegenerative disorders, there is a need for finding effective VDCCs blockers in order to control the levels of Ca²⁺ in nerve cells in order to obtain effective medicaments for the treatment of such diseases and disorders.

### SUMMARY OF THE INVENTION

A new family of compounds having VDCC blocking activity has been found. In a first aspect, the present invention is related to a compound of formula (I) 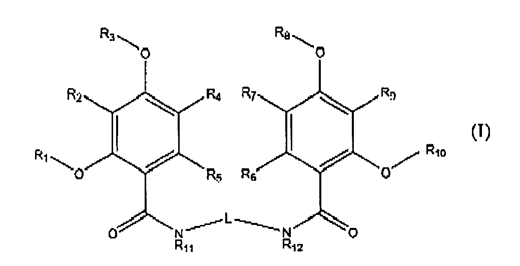 wherein
R₁ and R₁₀ are independently selected from hydrogen, substituted or unsubstituted alkyl or -CH₂ORₐ, wherein Rₐ is selected from hydrogen, substituted or unsubstituted alkyl, substituted or unsubstituted cycloalkyl, substituted or unsubstituted alkenyl, substituted or unsubstituted aryl, substituted or unsubstituted aralkyl or substituted or unsubstituted heterocyclyl;
R₃, R₈, R₁₁ and R₁₂ are independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, substituted or unsubstituted alkoxy, substituted or unsubstituted aryloxy or halogen;
R₂, R₄, R₅, R₆, R₇ and R₉ are independently selected from hydrogen or halogen, preferably Br;
L is a linker, consisting of a linear sequence of 1-20 units selected from -(CH₂)ₙ-, -CO-,-O-, -S-, substituted or unsubstituted arylene, cycloalkylene, heterocyclylene, or -NH-; n=1-10;
or its enantiomers, diastereomers, tautomers, and pharmaceutically acceptable solvates and salts thereof.

Due to their VDCC blocking activity, these compounds may be useful for the treatment of a series of human diseases and conditions, especially cognitive or neurodegenerative diseases or conditions; therefore, according to another aspect, the present invention is related to the use of a compound of formula (1) as defined above in the preparation of a medicament for the treatment of a cognitive or neurodegenerative disease or condition.

In the frame of the present invention, the term "cognitive or neurodegenerative disease or condition" should be interpreted as including, but not being limited to, stroke, ischemia, anxiety, epilepsy, head trauma, migraine, chronic pain, neuropathic pain and acute pain, schizophrenia, depression, psychoses, drug and alcohol addiction, and neurodegenerative disorders such as Parkinson's Disease, Alzheimer's Disease, multiple sclerosis, neuropathies, Huntington's Disease and amyotrophic lateral sclerosis (A.LS)).

A third aspect of the invention is a pharmaceutical composition which comprises at least one compound of formula (1) as defined above, or a pharmaceutically acceptable salt or solvate thereof, and a pharmaceutically acceptable carrier, adjuvant or vehicle.

The compounds of formula (I) according to the present invention may also be used as reactives for blocking VDCC in biological assays. Therefore, an additional aspect of the present invention is related to the use of the compounds of formula (I) as reactives for biological assays, preferably as reactives for blocking VDCC.

Another aspect of the present invention is a method of treating or preventing a disease or condition involving alterations of Ca²⁺ homeostasis, which method comprises administering to a patient in need of such a treatment a therapeutically effective amount of at least one compound of formula (I) as defined above or a pharmaceutical composition thereof.

### DETAILED DESCRIPTION OF THE INVENTION

In the above definition of compounds of formula (1) the following terms have the meaning indicated:
The term "alkenyl" refers to a linear, branched or cyclic hydrocarbon group of 2 to about 20 carbon atoms containing at least one double bond, such as ethenyl, n-propenyl, isopropenyl, n-butenyl, isobutenyl, octenyl, decenyl, tetradecenyl, hexadecenyl, eicosenyl, tctracosenyl, and the like. The term "substituted alkenyl" refers to alkenyl substituted with one or more substituent groups. The term "alkenyl" includes linear, branched, cyclic, unsubstituted, substituted, and/or heteroatom-containing alkenyl.
"Alkoxy" refers to a radical of the formula -ORa where Ra is an alkyl radical as defined below, c. g., methoxy, ethoxy, propoxy, etc.
"Alkyl" refers to a straight or branched hydrocarbon chain radical consisting of carbon and hydrogen atoms, containing no saturation, having one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted. by one or more substituents.
"Aralkyl" refers to an alkyl group with an aryl substituent, wherein "alkyl" and "aryl" are as defined above. In general, aralkyl groups herein contain 6 to 24 carbon atoms, while preferred aralkyl and alkaryl groups contain 6 to 16 carbon atoms, and particularly preferred such groups contain 6 to 12 carbon atoms. Examples of aralkyl groups include, without limitation, benzyl, 2-phenyl-ethyl, 3-phenyl-propyl, 4-phenylbutyl, 5-phenyl-pentyl, 4-phenylcyclohexyl, 4-benzylcyclohexyl, 4-phenylcyclohexylmethyl, 4-benzylcyclohexylmethyl, and the like. Alkaryl groups include, for example, p-methylphenyl, 2,4-dimethylphenyl, p-cyclohexylphenyl, 2,7-dimethylnaphthyl, 7-cyclooctylnaphthyl, 3-ehyl-cyclopenta-1,4-dienyl, and the like, preferably benzyl and phenethyl.
"Aryl" refers to an aromatic substituent generally containing 5 to 30 carbon atoms and containing a single aromatic ring or multiple aromatic rings that arc fused together, directly linked, or indirectly linked (such that the different aromatic rings are bound to a common group such as a methylene or ethylene moiety). Preferred aryl groups contain 5 to 24 carbon atoms, and particularly preferred aryl groups contain 5 to 14 carbon atoms. Exemplary aryl groups contain one aromatic ring or two fused or linked aromatic rings, e.g., phenyl, naphthyl, biphenyl, diphenylether, diphenylamine, benzophenone, indenyl, fenanthryl or anthracyl and the like. "Substituted aryl" refers to an aryl moiety substituted with one or more substituent groups. If not otherwise indicated, the term "aryl" includes unsubstituted, substituted, and/or heteroatom-containing aromatic substituents.
The term "arylene" refers to a diradical derived from aryl or substituted aryl as defined above, and is exemplified by 1,2-phenylene, 1,3-phenylene, 1,4-phenylene, 1,2-naphthylene and the like.
The term "aryloxy" refers to the group aryl-O- wherein the aryl group is as defined above including optionally substituted aryl groups as also defined above.
"Cycloalkyl" refers to a stable 3-to 10-membered monocyclic or bicyclic radical which is saturated or partially saturated, and which consist solely of carbon and hydrogen atoms. Unless otherwise stated specifically in the specification, the term "cycloalkyl" is meant to include cycloalkyl radicals which arc optionally substituted by one or more substituents.
"Cycloalkylene" refers to a diradical derived from cycloalkyl as defined above, being optionally substituted.
The terms "halo," "halide," and "halogen" refer to a chloro, bromo, fluoro, or iodo substituent.
The terms "heterocycle", "heterocyclyl", or "heterocyclic" refers to a stable 3-to 15 membered ring which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized ; and the heterocyclyl radical may be partially or fully saturated or aromatic. Examples of such heterocycles include, but arc not limited to, azepines, benzimidazole, benzothiazole, furan, isothiazole, imidazole, indole, piperidine, piperazine, purine, quinoline, thiadiazole, tetrahydrofuran.
"Heterocyclylene" refers to a diradical derived from heterocylyl as defined above; it may optionally be substituted by one or more substituents.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C1-C6 alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from I to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

In the above-detailed formula (I), R₃ and R₈ are preferably an alkyl, being the same or different. Even more preferably, R₃ and R₈ are methyl.

According to a preferred embodiment, the linker L consists of a linear sequence of 1 to 20 -(CH₂)- units.

According to another preferred embodiment, the linker L comprises an ether unit (-O-) subsequent to a substituted or unsubstituted arylene unit. Preferably, the arylene unit is a substituted or unsubstituted benzylene unit.

Also preferred are compounds of formula (1) wherein R₅ and R₆ are hydrogen.

Another group of preferred compounds are those wherein at least one of R₂, R₄, R₇ and R₉ is a halogen, preferably Br.

A further group of preferred compounds are those wherein R₁ is equal to R₁₀, R₂ is equal to R₉, R₃ is equal to R₈, R₄ is equal to R₇, R₅ is equal to R₆.

Preferably, also the linker L is symmetric, the compounds having a symmetry plane.

The following are preferred compounds of formula (I) according to the present invention:

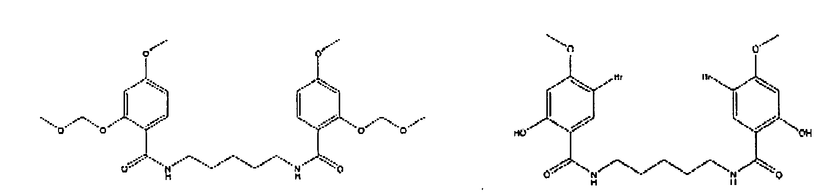

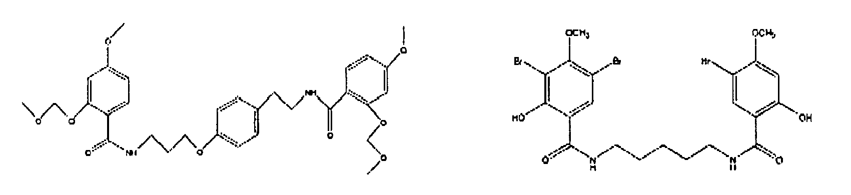

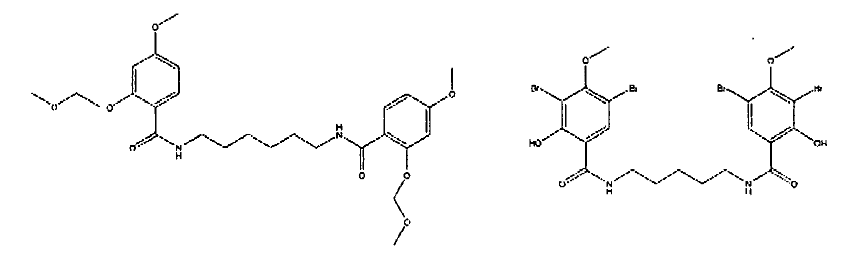

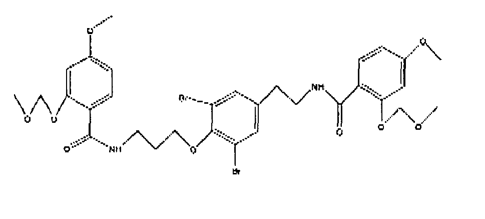

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a 13C- or 14C-cnriched carbon or 15N-enriched nitrogen are within the scope of this invention.

The term "pharmaceutically acceptable salts or solvates" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts and derivatives can be carried out by methods known in the art.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluencsulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

In another aspect, the present invention is referred to a compound of formula (I) as defined above, for use as a medicament.

A further aspect of the invention is the use of a compound of formula (T) as defined above in the preparation of a medicament for the treatment of a cognitive or neurodcgcnerative disease, disorder or condition.

In the frame of the present invention, the term "cognitive or neurodegenerative disease or condition" should be interpreted as including, but not being limited to, stroke, ischemia, anxiety, epilepsy, head trauma, migraine, chronic pain, neuropathic pain and acute pain, schizophrenia, depression, psychoses, drug and alcohol addiction, and neurodegenerative disorders such as Parkinson's Disease, Alzheimer's Disease, multiple sclerosis, neuropathies, Huntington's Disease and amyotrophic lateral sclerosis (ALS)). Anxiety includes but is not limited to panic disorder, obsessive-compulsive disorder and post-traumatic stress syndrome; chronic pain includes but is not limited to cancer pain, inflammatory pain conditions related to osteoarthritis, rheumatoid arthritis and fibromyalgia; neuropathic pain includes but is not limited to diabetic peripheral neuropathy, post-herpetic neuralgia, trigeminal neuralgia, cancer pain and AIDS related neuropathy; acute pain includes but is not limited to nociceptive pain and post-operative pain.

More preferably, the cognitive or neurodegenerative disease, disorder or condition is Alzheimer's Disease. In another embodiment the disease or condition is epilepsy.

According to another aspect of the present invention, it is referred to a pharmaceutical composition which comprises at least one compound of formula (1) as defined above or a pharmaceutically acceptable salt or solvate thereof, and a pharmaceutically acceptable carrier, adjuvant or vehicle.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopocias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of many of the diseases to be treated.

Generally an effective administered amount of a compound of the invention will depend on the relative efficacy of the compound chosen, the severity of the disorder being treated and the weight of thc sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Taking into account that the compounds of formula (I) exhibit an inhibitory effect on VDCCs, the compounds may be used as reactives for biological assays, especially as reactives for blocking VDCCs. Therefore, another aspect of the invention is the use of a compound of formula (I) as defined above, or any salt or solvate thereof, as reactives for biological assays, preferably as a reactive for blocking VDCC.

A further aspect of the invention is a method of treating or preventing a disease, disorder or condition involving alterations of Ca²⁺ homeostasis, which method comprises administering to a patient in need of such a treatment a therapeutically effective amount of a compound of formula (I) as defined above, or any salt or solvate thereof, or a pharmaceutical composition thereof.

Final compounds of formula (I) according to the present invention can be obtained by a convergent pathway strategy which consist on coupling the conveniently substituted benzoic acid intermediate to the corresponding alkylic or arylic amines employing the methodology previously described by Padwa, A. et al, Synthesis, 1994, 9, 993-1004. The benzoic acid intermediate was obtained following synthetic standard procedures widely reported in the literature. While alkylic diamines are commercially available from Sigma-Aldrich the arylic amines were obtained from tyramine following similar reported literature procedures (Schoenfeld, R. C.; Conova, S.; Rittschof, D. and Ganem, B. Bioorganic & Medicinal Chemistry Letters 2002, 12, 823-825).

The following examples are given as further illustration of the invention, they should in no case be taken as a definition of the limits of the invention.

### EXAMPLES

### PREPARATION OF THE COMPOUNDS

Compounds of formula (I) according to the present invention were prepared following the general preparation strategy detailed above. Concretely, 7 compounds, named within this invention compounds 1 to 7, with structures as detailed in table 1, were synthesized.

**Table 1**

| **Compound No.** | **Structure** |
|---|---|
| **1** | 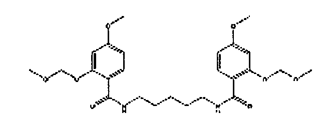 |
| **2** | 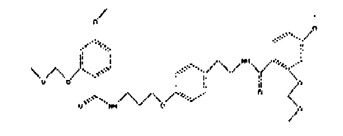 |
| **3** | 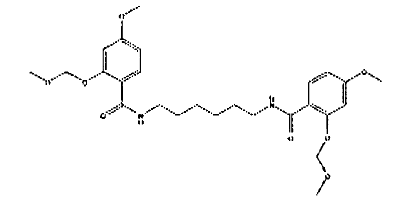 |
| **4** | 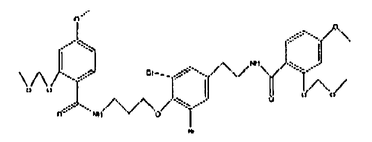 |
| **5** | 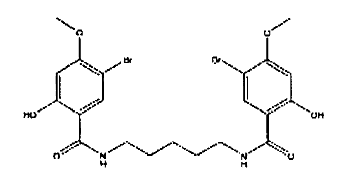 |
| **6** | 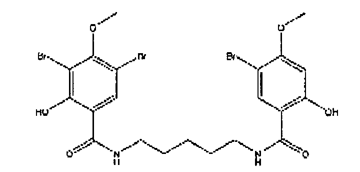 |
| **7** | 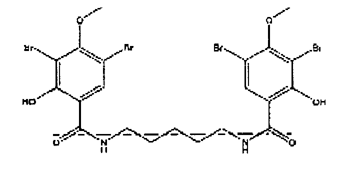 |

The compounds **1 - 4** were prepared according to the following general method:
To a solution of 2-(methoxymethoxy)-4-(methoxy) benzoic acid in anhydrous THF, 1,1'-carbollyldiimidazol was added under N₂ atmosphere, and the resulting mixture was stirred for four hours at room temperature. Afterwards, a solution of the corresponding diamines in anhydrous THF (DMF was also added when the corresponding diamine was not soluble in THF), and TEA (2 eq, only when the diamine was used as its trifluoroacetic salt) was added and the reaction mixture was stirred for further 20 hours. After evaporation of the solvent under reduced pressure, water was added and the resulting mixture was extracted with DCM. The combined organic extracts were washed with saturated NaCl solution and dried with Na₂SO₄. Evaporation of the solvent under reduced pressure gave a residue which was purified by silica gel flash-column chromatography as indicated below for each case, giving compounds **1-4.** (see Scheme 1)

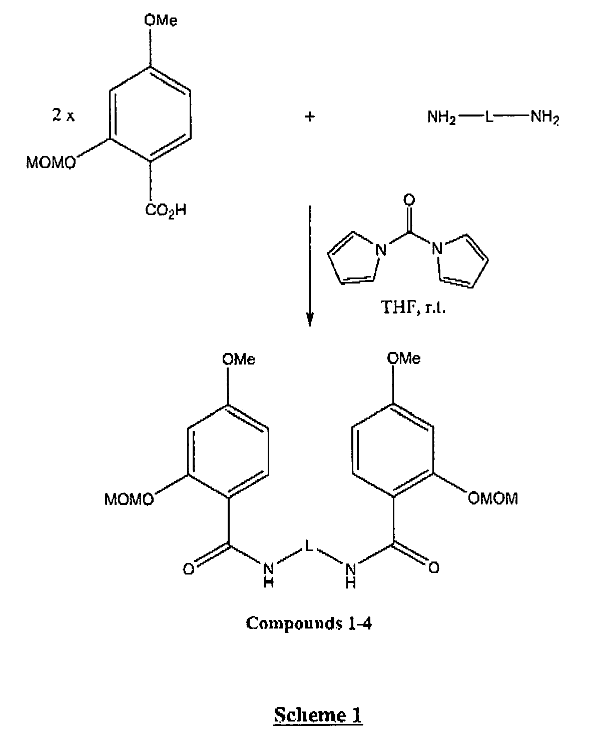

The intermediates which are necessary for this general procedure may be prepared as follows:

### Synthesis of the intermediate 2-(methoxymethoxy)-4-(methoxy) benzoic acid

A mixture of2-hydroxy-4-methoxy-benzoic acid (5.0 g, 29.3 mmol) in MeOH (150 mL) and H₂SO₄ (2 mL) was refluxed for 48 hours. After evaporation of the solvent to reduced pressure, DCM (100 mL) was added and the solution was washed with water (100 mL), 10 % K₂CO₃ solution, saturated NaCl solution and subsequently dried (Na₂SO₄), to give 4.8 g of the 2-hydroxy-4-methoxy-benzoic acid methyl ester derivative (89 %) as a white solid.

A solution of this compound (4.8 g, 26.2 mmol) in anhydrous THF (24 mL) at 0 °C was treated with DIPEA (5.76 mL, 32.9 mmol) and subsequently with methoxymetllyl chloride (2.47 mL, 32.9 mmol) over a period of 10 minutes. The reaction mixture was left to further stir at room temperature for 24 hours. Diethyl ether (200 mL) was added and the resulting solution was washed with water (2 x 100 mL), 0.1 M HCl solution (2 x 100 mL) and subsequently dried (Na₂SO₄), to give a residue which was purified by column chromatography (eluent used; hexane:ethyl acetate from 10:1 to 5:1), to give 5.9 g (88 %) 2-(methoxymethoxy)-4-(methoxy) benzole acid methyl ester.

The latter (4.4 g, 19.6 mmol) was hydrolysed by treatment with lithium hydroxide monohydrate (4.1 g, 97.9 mmol) in water/THF 1:3 (150 mL) for 3 days. THF was evaporated and the water phase cooled in an ice-bath, was neutralised to pH 3-4 with 0.1 M HCl solution, and extracted with DCM (4 x 50 mL). The combined extracts were dried (Na₂SO₄) and the solvent evaporated, to give 3.5 g (85%) of the 2-(methoxymethoxy)-4-(methoxy) benzoic acid as a white solid.

### Synthesis of the arylic diamine intermediates

### Synthesis of 3-[4-(2-aminoethyl)-phenoxy]-propylamine diacetate salt

To a solution of tyramine (4-(2-aminoethyl)-phenol) (2.0 g, 14.6 mmol) in anhydrous DCM (30 mL), TEA (4.06 mL, 29.2 mmol) was added at room temperature. BOC anhydride (1.9g, 8.76 mmol) was slowly added at 0 °C and the resulting mixture was stirred at room temperature for 2 days. DCM (50 mL) was added and the organic phase was washed with 0.1 M HCl (50 mL), water (3 x 100 mL), saturated NaCl solution, and subsequently dried (Na₂SO₄), and the solvent evaporated under reduced pressure, providing 2.15 g (61 %) of [2-(4-hydroxy-phenyl)-ethyl]-carbamic acid *tert*-butyl ester.

A mixture of the above *tert*-butyl ester derivative (15.6 g, 66.0 mmol), N-(3-bromopropyl)-phtalimide (12.7 g, 66.0 mmol), K₂CO₃ (22.8 g, 132 mmol) and KI (3.29 g, 19.8 mmol) in acetonitrile was refluxed for 24 hours. The solvent was evaporated to dryncss, water was added (300 mL), and the resulting mixture was extracted with DCM (2 x 300 mL). The combined extracts were washed with saturated NaCl solution, dried (Na₂SO₄) and the solvent removed. The resulting product was triturated in acetonitrile and filtered to give 20.2 g (72%) of (2-{4-[3-(1,3-dioxo-1,3-dihydroisoindolo-2-yl)propoxy]-phenyl-ethyl)-carbamic acid *tert*-butyl ester.

A mixture of the above compound (20.2 g, 48 mmol) with hydrazine monohydrate (6.8 mL, 140 mmol) in MeOH (400) was refluxed for 4 hours. After evaporation of the solvent, the white solid obtained was suspended in DCM and the mixture cooled in an ice-bath. Filtration of the white precipitate gave 11.53 g (86 %) of 3-[4-(2-amino-propoxy)-phenylamino]-propionic acid *tert*-butyl ester.

Treatment of this compound (1.9 g, 7.0 mmol) with TFA (25 mL) in THF (75 mL) at room temperature for 24 hours gave 2.80 g (93 %) of 3-[4-(2-aminoethyl)-phenoxy]-propylamine as diacetate salt.

### Synthesis of 3-[4-(2-aminoethyl)-2,6-dibromo-phenoxy]-propylamine diacetate salt

It was synthesised from 4-(2,6-dibromo-2-aminoethyl)-phenol employing the same methodology described above. 4-(2,6-Dibromo-2-aminoethyl)-phenol was obtained by bromination of 4-(2-aminoethyl)-phenol. Thus, a solution of 4-(2-aminoethyl)-phenol (2 g, 14.6 mmol) in CHCl₃ (80 mL) was treated with pyridinium tribromide (9.3 g, 29.19 mmol) in pyridine (21 mL) for 24 hours and the solvent was evaporated to dryness. The brown solid obtained was suspended in water and cooled into an ice-bath. The precipitated was filtered and dried to give 4.14 g (98 %) of 4-(2,6-dibromo-2-aminoethyl)-phenol. Structural characterization data are in accordance with that in the literature (Scheuer, P. J. and Hamann, M. T., J. Org. Chem. 1993, 58, 6565-6569).

### Example 1

### Preparation of compound 1:

Reagents: 2-(methoxymethoxy)-4-(methoxy)-benzoic acid (150 mg, 0.71 mmol) in anhydrous THF (5 mL); 1,1'-carbonyldiimidazol (120 mg, 0.74 mmol); 1,5-diaminopentane (50 µl, 0.42 mmol) in THF (5 mL).
Purification: silica gel flash-chromatography using EtOAc:McOH (20:1).
Yield: 28 mg (16%) as a white solid.
¹H-NMR (CDCl₃, 400MHz, δ ppm): (8.10, d, 2H, *J*=8.6 Hz), (7.71, brs, 2H, NH), (6.60, d, 2H, *J*=2.3 Hz), (6.64, dd, 2H, *J*=2.3 Hz, *J*=8.6 Hz), (5.24, s, 4H), (3.79, s, 6H), (3.46, s, 6H), (3.43, m, 4H), (1.64, m, 4H), (1.47, m, 2H).
¹³C-NMR (CDCl₃, 100 MHz, δ ppm): 165.1, 163.1, 156.5, 133.7, 115.3, 107.0, 101.3, 95.2, 56.8, 55.6, 39.6,29.5,24.6.
ES1-MS[M+H]⁺491.01.

### Example 2

### Preparation of Compound 2

Reagents: 2-(methoxymethoxy)-4-(methoxy)-benzoic acid (3.4 g, 15.8 mmol), anhydrous THF (20 mL); 1,1'-carbonyldiimidazol (2.7 g, 16.6 mmol); 3-[4-(2-aminoethyl)-phenoxy]-propylamine diacetate salt (4.0 g, 9.5 mmol) and TEA (4.6 mL, 5.17 mmol) in THF/DMF(12 mL, 1:1).
Purification: was not required.
Yield: 4.6 g (84%) as white solid.
¹H-NMR (CDCl₃, 400MHz, δ ppm): (8.14, t, 2H, *J*=5.4 Hz), (8.00, t, 2H, *J*=5.4 Hz), (7.79, d, 1H, *J*-8.8 Hz), (7.73, d, 1H, *J*=8.8 Hz), (7.17, d, 2H, *J*=8.0 Hz), (6.88, d, 2H, *J*=8.0 Hz), (6.69, t, 2H, *J*=5.2 Hz), (6.65, s, 2H), (5.29, s, 2H), (5,25, s, 2H), (4.01, d, 2H, *J*=5.6 Hz), (3.77, s, 6H), (3.50-3.46, m, 4H), (3.35, s, 3H), (3.30, s, 3H), (2.76, t, 2H, *J*=6.8 Hz), (1.94, m, 2H).
¹³C-NMR (CDCl₃, 100 MHz, δ ppm): 164.56, 164.2, 162.2, 162.1, 157.08, 155.9, 155.8, 132.2, 131.9, 131.3, 129.5, 116.6, 115.9, 114.4, 106.9, 106.8, 101.3, 101.2, 94.6, 94.4, 65.5, 56.0, 56.0, 55.4, 40.6, 36.2, 34.1, 30.6, 28.9. ESI-MS[M]⁺ 582.9.

### Example 3

### Preparation of Compound 3

Reagents: 2-(methoxymethoxy)-4-(methoxy)-benzoic acid (500 mg, 2.3 mmol) in anhydrous THF (10 mL); 1,1'-carbonyldiimidazol (400 mg, 2.5 mmol); 1,6-diaminohexane (164 mg, 1.41 mmol) in THF (4 mL).
Purification: silica gel flash-chromatography using EtOAc:MeOH (100:1).
Yield: 426 mg (72%) as a white solid.
¹H-NMR (CDCl₃, 400MHz, δ ppm): (8.12, d, 2H, *J*=9.0 Hz), (7.69, brs, 2H, NH), (6.65, d, 2H, *J*=2.3 Hz), (6.61, dd, 2H, *J*=2.3 Hz, *J*=9.0 Hz), (5.27, s, 4H), (3.80, s, 6H), (3.49, s, 6H), (3.42, m, 4H), (1.68-1.58, m, 4H), (1.44-1.41, m, 4H).
¹³C-NMR (CDCl₃, 100 MHz, δ ppm): 165.1, 163.1, 156.5, 133.7, 115.6, 107.2, 101.5, 95.4, 56.8, 55.6, 39.7, 29.8, 26.9.
ESI-MS[M]⁺505.05.

### Example 4

### Preparation of Compound 4

Reagents: 2-(methoxymethoxy)-4-(methoxy)-benzoic acid (500 mg, 2.3 mmol), anhydrous THF (10 mL); 1,1'-carbonyldiimidazol (400 mg, 2.5 mmol); 3-[4-(2-aminocthyl)-2,6-dibromo-phenoxy]-propylamine diacetate salt (817 mg, 1.4 mmol) and TEA (0.7 mL, 5.17 mmol) in THF/DMF (11 mL, 10:1).
Purification: EtOAc:hexane (4:1).
Yield: 547 mg (63%) as white solid.
¹H-NMR (CDCl₃, 400MHz, δ ppm): (8.15, d, 1H, *J*=6.3 Hz), (8.14, brs, 1H), (8.13, d, 1H, *J*=6.3 Hz), (7.73, brs, 1H), (7.40, s, 2H), (6.69, d, 1H, *J*=2.4 Hz), (6.66, d, 1H, *J*=2.4 Hz), (6.64-6.60, m, 2H), (5.17, s, 2H), (5.16, s, 2H), (4.12-4.07, m, 2H), (3.81, s, 6H), (3.78, q, 2H, *J*=6.2 Hz), (3.67, q, 2H, *J*=5.8 Hz), (3.38, s, 3H), (3.35, s, 3H), (2.84, t, 2H, *J*=6.8 Hz), (2.15, m, 2H).
¹³C-NMR (CDCl₃, 100 MHz, δ ppm): 171.1, 165.1, 165.0, 163.3, 163.0, 156.7, 156.5, 151.5, 138.5, 133.6, 133.0, 118.2, 115.3, 114.8, 107.1, 107.0, 101.4, 95.2, 95.0, 60.3, 56.5, 56.3, 55.5, 55.5, 40.5, 37.5, 34.6, 29.9, 14.2.
EST-MS[M]⁺ 740.9.

The Compounds 5-7 were prepared starting from Compound **1**, according to the following procedure:
Compound **1** (176 mg, 0.4 mmol) in DCM (5 mL) was dropwise added to a solution of pyridinium tribromide in pyridine (2 mL) at 0 °C, and then the reaction mixture was left to stir at room temperature for 20 hours. The resulting reaction mixture was diluted with DCM (50 mL) and washed with water (50 mL), 3M HCl solution (50 mL) and saturated NaCl solution (50 mL). The organic extract was dried (Na₂SO₄) and the solvent evaporated under reduced pressure giving a white solid which after purification by flash-column chromatography (eluent; DCM:MeOH, 200:1) gave a residue containing a mixture of compounds 5-7. Compounds 5, 6 and 7 were successfully separated by preparative HPLC obtaining 13 mg (7%), 23 mg (10%) and 2 mg (0.7%) respectively. (see Scheme 2)

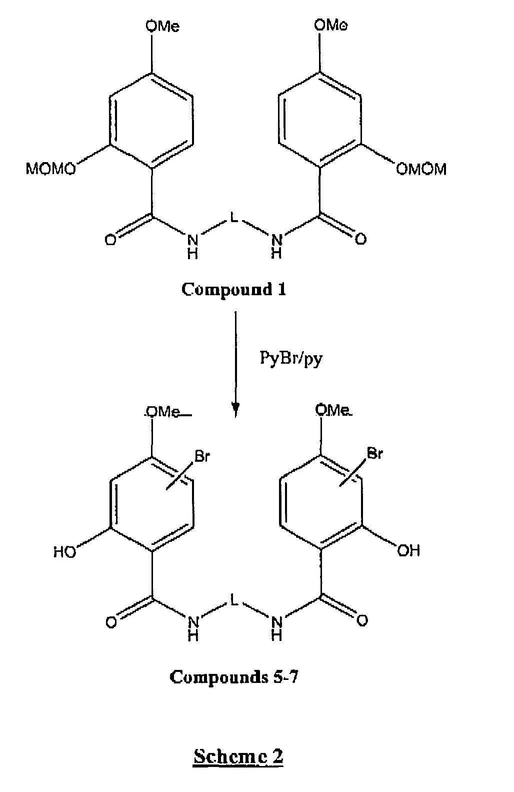

### Example 5

### Compound 5

¹H-NMR (Acetone-d₆, 400MHz, δ ppm): (8.12, brs, 2H, NH), (7.94, s, 2H), (6.54, s, 2H), (3.91, s, 6H), (3.41, m, 4H), (1.69-1.66, m, 4H), (1.49-1.47, m, 2H).
¹³C-NMR (Acetone-d₆, 100 MHz, δ ppm): 169.2, 160.0, 130.6, 108.7, 101.2, 99.9, 56.1. 39.3, 29.9, 24.2.
ESI-MS[M]⁺561.

### Example 6

### Compound 6

¹H-NMR (CDCl₃, 400MHz, δ ppm): (7.59, s, 1H), (7.5o, s, 1H), (6.54, s, 2H), (6.20, brs, 2H, NH), (3.91, s, 3H), (3.88, s, 3H), (3.44, m, 4H), (1.69-1.66, m, 4H), (1.49-1.47, m, 2H),
¹³C-NMR (CDCl₃, 100 MHz, δ ppm): 169.0, 168.2, 162.9, 160.1, 159.2, 158.5, 129.4, 128,4, 112.6, 108.7, 108.2, 106.1, 101.4, 100.6, 60.7, 56.4, 39.8, 39.4, 29.1, 28.9, 24.1.
ESI-MS[M]⁺640.

### Example 7

### Compound 7

¹H-NMR (CDCl₃, 400MHz, δ ppm): (7.49, s, 2H), (6.27, brs, 2H, NH), (3.85, s, 6H), (3.40, m, 4H), (1.69-1.66, m, 4H), (1.49-1.47, m, 2H).
¹³C-NMR (CDCl₃, 100 MHz, δ ppm):168.23, 1590.30, 158.7, 128., 112.6, 101.85, 106.2, 60.7, 39.7, 29.0, 24.0.
ESI-MS[M]⁺719.

### BIOLOGICAL ACTIVITY OF THE COMPOUNDS OF FORMULA (I)

### Example 8: VDCC Inhibition of the compounds

This assay is aimed to determine the VDCC blocker activity of compounds; it is performed using SH-SY5Y neuroblastoma cells. SH-SY5Y cells were plated at 5x10⁵ cells per well into Black / Clear Bottom 96-well culture plate, 48 hours before treatment. Cells were loaded with Fluo-4, 5 µM and pluronic acid, 0.1%, for 30 min at 37°C, 5%CO₂, following a incubation of 15 min at RT in Krebs-HEPES solution. Immediately cells are exposed to the samples at different concentrations for 10 min. The compounds were tested at 10⁻⁵ to 10⁻⁵ M, depending on potency. After the treatment, calcium entry is measured as fluorescence in a Fluostar Optima plate reader (BMG) in response to depolarization with 60 mM KCl. The excitation wavelength was 485 nm, and that of emission 520 nm.

The compounds of formula (1) according to the present invention showed VDCC blocker activity. Results are shown in table 2.

**Table 2**

| Compound No. | Structure | % of calcium entry inhibition | | | |
|---|---|---|---|---|---|
| | | 10⁻⁵ | 10⁻⁶ | 10⁻⁷ | 10⁻⁸ |
| 1 | 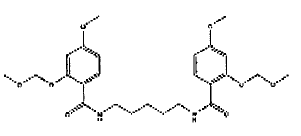 | 57,8 ± 16 | / | / | / |
| 2 | 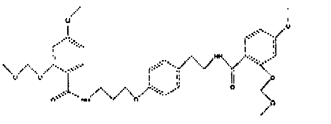 | 93,3 ± 5 | 34,2 ± 1.5 | 29,7 ± 14 | 31,2 ± 9 |
| 3 | 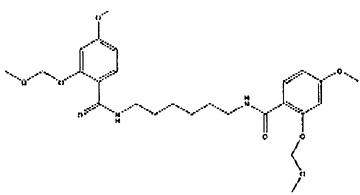 | 37.5 ± 7 | 6,6 ± 2 | / | / |
| 4 | 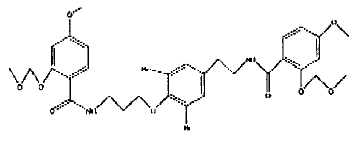 | 87.3 ± 5 | 27.3 ± 5 | 6 ± 0 | / |
| 5 | 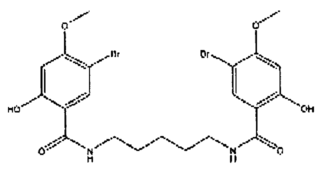 | 21.8 ± 5 | / | / | / |
| 6 | 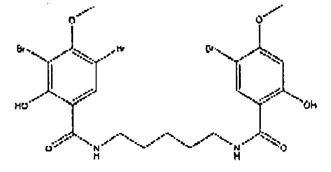 | 96.6 ± 4 | 48.7 ± 18 | 27.5 ± 5 | 11.8 ± 16 |
| 7 | 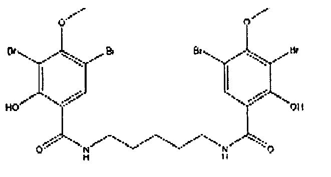 | 90.7 ± 3 | 3,1 ± 0 | / | / |

### Example 9: Toxicity measurement

The cytotoxicity effect of the molecules was tested in the human neuroblastoma cell line SH-SY5Y. These cells were cultured in 96-well plates in minimum essential medium, Ham's F12 medium, supplemented with 10% fetal bovine serum, 1% glutamine and 1% penicillin/streptomycin, and grown in a 5% CO₂ humidified incubator at 37°C. Cells were plated at 10⁴ cells for each well, at least, 48 hours before treatment. Cells were exposed for 24 hours to the compounds at different concentrations, quantitative assessment of cell death was made by measurement of the intracellular enzyme lactate dehydrogenase (LDH) (citotoxicity detection kit, Roche). The quantity of LDH was measured was evaluated in a microplate reader Dygiscan (Asys Hitech GmbH), at 492 and 620 um. Controls were taken as 100% viability. All compounds 1 to 7 were tested for toxicity at a concentrations of 10⁻⁵ and 10⁻⁶ M, and resulted as non-toxic.

## Claims

1. A compound of formula (I) 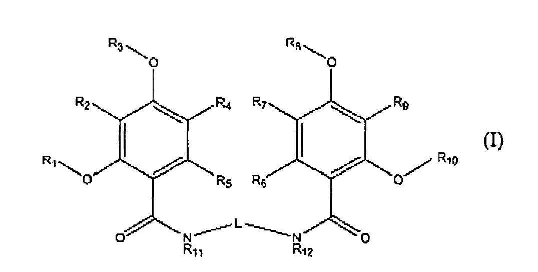 wherein
R₁ and R₁₀ are independently selected from hydrogen, substituted or unsubstituted alkyl or -CORₐ, wherein Rₐ is selected from hydrogen, substituted or unsubstituted alkyl, substituted or unsubstituted cycloalkyl, substituted or unsubstituted alkenyl, substituted or unsubstituted aryl, substituted or unsubstituted aralkyl or substituted or unsubstituted heterocyclyl;
R₃, R₈, R₁₁ and R₁₂ are independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, substituted or unsubstituted alkoxy, substituted or unsubstituted aryloxy or halogen;
R₂, R₄, R₅, R₆, R₇ and R₉ arc independently selected from hydrogen or halogen, preferably Br;
L is a linker, consisting of a linear sequence of 1-20 units selected from -(CH₂)ₙ-, -CO-,-O-, -S-, substituted or unsubstituted arylene, cycloalkylene, heterocyclylene, or -NH-; n = 1-10;
or its enantiomers, diastereomers, tautomers, and pharmaceutically acceptable solvates and salts thereof.

2. A compound according to claim 1, wherein R₃ and R₈ arc independently an alkyl.

3. A compound according to claim 2, wherein R₃ and R₈ are methyl.

4. A compound according to any one of claims 1 to 3, wherein linker L consists of a linear sequence of 1-20 -(CH₂)- units.

5. A compound according to any one of claims 1 to 3, wherein linker L comprises a -0- unit subsequent to a substituted or unsubstituted arylene unit.

6. A compound according to claim 5, wherein the arylene unit is a substituted or unsubstituted benzylene unit.

7. A compound according to any one of the previous claims, wherein R₅ and R₆ are hydrogen.

8. A compound according to any one of the previous claims, wherein at least one of R₂, R₄, R₇ and R₉ is a halogen, preferably Br.

9. A compound according to any one of the previous claims, wherein R₁ is equal to R₁₀, R₂ is equal to R₉, R₃ is equal to R₈, R₄ is equal to R₇, R₅ is equal to R₆.

10. A compound according to claim 9, wherein linker L is symmetric, the compound having a symmetry plane.

11. A compound as defined in claim 1 selected from:

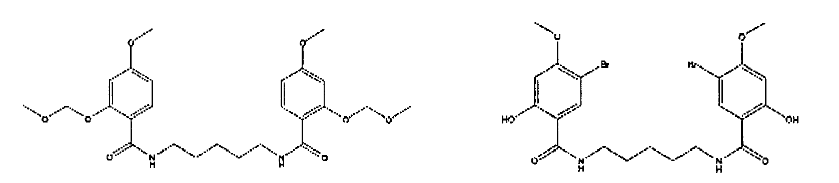

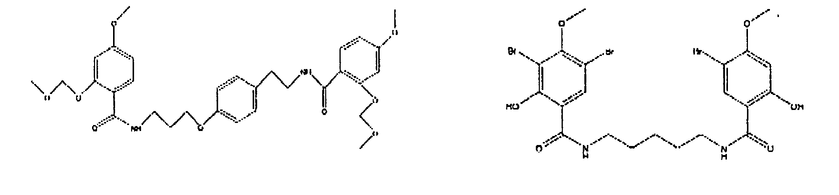

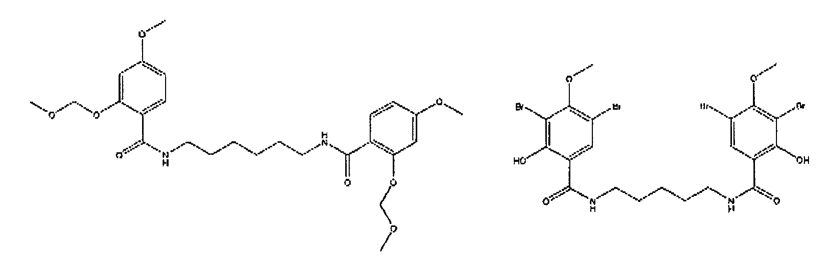

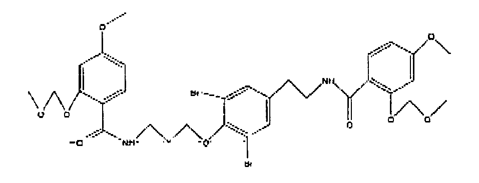

12. Compound of formula (I) as defined in any of claims 1 to 11 for use as a medicament.

13. Use of a compound of formula (I) as defined in any of claims 1 to 11 in the preparation of a medicament for the treatment of a cognitive or neurodegenerative disease.

14. Use of a compound according to claim 13, wherein the cognitive or neurodegenerative disease is selected from stroke, ischemia, anxiety, epilepsy, head trauma, migraine, chronic pain, neuropathic pain and acute pain, schizophrenia, depression, psychoses, drug and alcohol addiction, and neurodegenerative disorders such as Parkinson's Disease, Alzheimer's Disease, multiple sclerosis, neuropathies, Huntington's Disease and amyotrophic lateral sclerosis (ALS).

15. Use of a compound according to claim 14, wherein the neurodegenerative disease is Alzheimer's Disease.

16. Use of a compound according to claim 14, wherein the disease or condition is epilepsy.

17. A pharmaceutical composition which comprises at least one compound of formula (I) as defined in any one of claims 1 to 11 or a pharmaceutically acceptable salt, or solvate thereof, and a pharmaceutically acceptable carrier, adjuvant or vehicle.

18. Use of a compound of formula (I) as defined in any of claims 1 to 11 as reactives for biological assays, preferably as a reactive for blocking VDCC.

19. Method of treating or preventing a disease or condition involving alterations of Ca²⁺ homeostasis, which method comprises administering to a patient in need of such a treatment a therapeutically effective amount of at least one compound of formula (1) as defined in any of claims 1 to 11 or a pharmaceutical composition thereof.
